Europäisches Patentamt

European Patent Office

Office européen des brevets

(19)

(11) Veröffentlichungsnummer: **0 195 444**
**A2**

(12) ## EUROPÄISCHE PATENTANMELDUNG

(21) Anmeldenummer: **86103784.4**

(22) Anmeldetag: **20.03.86**

(51) Int. Cl.⁴: **A 61 B 17/22**

(30) Priorität: **22.03.85 DE 3510517**

(43) Veröffentlichungstag der Anmeldung:
**24.09.86 Patentblatt 86/39**

(84) Benannte Vertragsstaaten:
**AT BE CH DE FR GB IT LI LU NL SE**

(71) Anmelder: **Körber, Günther**
**Berliner Strasse 32**
**D-8898 Schrobenhausen(DE)**

(72) Erfinder: **Körber, Günther**
**Berliner Strasse 32**
**D-8898 Schrobenhausen(DE)**

(54) **Vorrichtung zum Einfangen und Entfernen von Uretersteinen oder Nierensteinen im Nierenbecken oder im Harnleiter.**

(57) Die Erfindung bezieht sich auf eine Vorrichtung zum Einfangen und Entfernen von Uretersteinen oder Nierensteinen im Nierenbecken oder im Harnleiter. Es handelt sich hierbei um einen Schlingenkatheter, bei dem das Rohr (1) Abflachungen (2, 3) sowie Schlitze (4) besitzt. Nach dem Vorbeiführen am Stein (8) wird die Schlinge zu einem käfigartigen Gebilde geschlossen, das die Besonderheit hat, den Stein (8) flächig (7, 2) zu umfassen und dadurch die Sicherheit gegen Abrutschen am Stein wesentlich zu erhöhen. Gleichzeitig wird durch die Abflachung (2) von einem größeren Flächenteil des Steins (8) die Schleimhaut gelöst und die Beweglichkeit des Steins im Harnleiter erleichtert.

Anders als bei bekannten käfigartigen Extraktoren bringt die erfindungsgemäße Schlinge formbedingt beim Einführen und Einfangen des Steins eine geringere Verletzungsgefahr des Harnleiters mit sich.

Fig. 3

EP 0 195 444 A2

## Vorrichtung zum Einfangen und Entfernen von Ureter- steinen oder Nierensteinen im Nierenbecken oder im Harnleiter

Auf dem Weltmarkt werden von einer begrenzten Anzahl von Fachfirmen mehrere in der Praxis angewendete Fanggeräte für Uretersteine bzw. Nierensteine angeboten, die sich im Harnleiter bzw. im Austrittstrichter der Niere befinden. Bekannt sind z.B. die Zeiss-Schlinge und Verbesserungen davon, wie DE-GM 77 36 691. Hierbei wird nach Vorbeiführen eines Katheterrohres von 5 bis 7 Charr. am Ureterstein mittels Fadenzug aus diesem eine Schlinge geformt, die den Stein umfaßt.

Es sind Entwicklungen bekannt, die dadurch gekennzeichnet sind, daß sie Käfige verschiedener Art bilden, wie z.B. der Hammel-Extraktor oder der Extraktor gemäß DE-PS 30 39 174. Diese bekannten Vorrichtungen haben jedoch den Nachteil, daß damit zwar der Stein im Käfig gehalten werden kann, aber beim Einführen in den Harnleiter, beim Einfangen des Steines und Herausziehen erhebliche Verletzungen der Schleimhaut des Harnleiters verursacht werden, da die rauhe Steinoberfläche durch den Käfig nicht ausreichend abgedeckt wird.

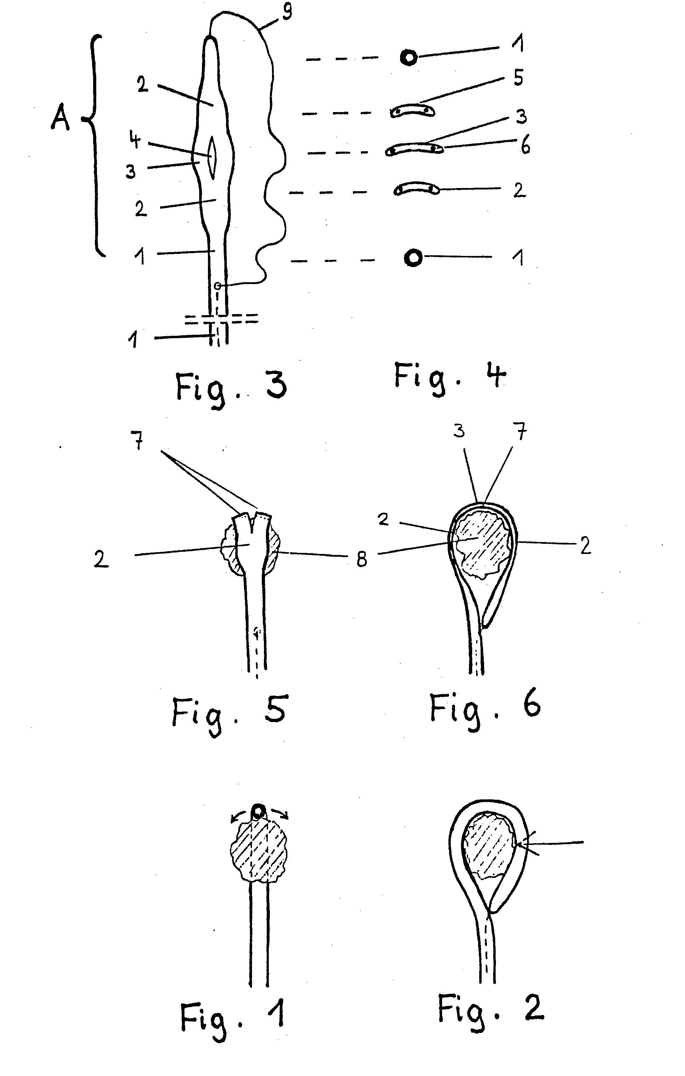

Anstelle der sogenannten Käfige werden deshalb heute in der Praxis vorwiegend Schlingen nach Fig. 1 und 2 eingesetzt, bei denen die Verletzungsgefahr des Harnleiters kleiner ist.

Diese Schlingen bestehen aus Kunststoffrohren, die nach dem Vorbeiführen am Stein über ein bekanntes Verfahren durch einen Faden zu einer Schlinge nach Fig. 1 und 2 geformt werden.

Da diese Schlingen über ihre volle Länge aus einem
Rohrquerschnitt bestehen, haben diese Schlingen den
Nachteil, daß sie nur punktförmig bzw. linienförmig
den Stein berühren, d.h. nur ein kleiner Bereich der
Steinoberfläche abgedeckt wird, und deshalb rutschen
solche Schlingen nach Äußerung von namhaften Urologen
bei 40 - 50 % der ärztlichen Eingriffe ab. Bei rundförmigen Steinen müssen diese herkömmlichen Schlingen
sogar 3 bis 4 Mal gelegt werden. Zwischen den einzelnen
Eingriffen vergehen jeweils mehrere teure und für den
Patienten unangenehme Krankenhaustage.

Aufgabe der Erfindung ist es, die Abrutschgefahr der
Schlingen erheblich zu vermindern und somit auch
kürzere Behandlungszeiten der Patienten zu erreichen.

Diese Aufgabe wird bei einer Vorrichtung zum Einfangen
und Entfernen von Ureter- oder Nierensteinen gemäß der
Erfindung dadurch gelöst, daß der Endteil A der
Schlinge auf wenigstens einem Teil seiner Länge eine
Breitenabmessung 2,3 hat, die größer als der Außendurchmesser des Rohres 1 ist. Die größere Breitenabmessung 2, 3 wird durch Abflachung des Rohres 1
gebildet. In dem abgeflachten Teil befinden sich
ein oder mehrere Schlitzöffnungen 4, die sowohl in
Längs- als auch in Querrichtung angeordnet sein können.
Diese abgeflachte größere Breitenabmessung 2,3 hat
den Vorteil, daß die Schlinge im geschlossenen
Zustand den Stein 8 flächenhaft hält (Fig. 5). Durch
die flächenhafte Berührung 7, die durch die Schlitze 4
so ausgebildet wird, daß die Berührungsflächen 7
schräg zueinander geneigt sind, wird ein Abrutschen
des Steins 8 verhindert. Die abgeflachte größere
Breitenabmessung 2 hat den Vorteil, daß sie die
Schleimhaut über eine größere Fläche vom Stein
ablöst und so die Beweglichkeit des Steins im Harnleiter verbessert. Eine Krümmung der abgeflachten größeren Breitenabmessung 2, 3 verbessert die Umfassung des Steins und vermindert so die Gefahr des Abrutschens. Durch die fließenden Übergänge zwischen dem Rohr 1 und der abgeflachten größeren Breitenabmessung 2, 3 besteht nur eine sehr geringe Verletzungsgefahr des Harnleiters.

Zweckmäßige Ausgestaltungen der Erfindung sind in den Unteransprüchen angegeben.

Die Erfindung wird anhand der beiliegenden Zeichnung
näher erläutert. Es zeigen

Fig. 1      Schnitt durch eine herkömmliche Schlinge
            mit dem Nachteil, daß sie nur punktförmig
            am Ureterstein aufliegt und deshalb erfahrungsgemäß seitlich leicht abrutscht;

Fig. 2      Ansicht einer herkömmlichen Schlinge, mit
            dem Nachteil der punktförmigen Berührung
            mit dem Ureterstein;

Fig. 3      Ansicht Endteil (A) des Rohres (1) mit
            größerer Breitenabmessung (2, 3), Schlitzöffnung (4) und Faden (9) zum späteren
            Schließen der Vorrichtung;

Fig. 4      Schnitt durch das Endteil (A) im Bereich
            des Rohres (1), im Bereich der größeren
            Breitenabmessung (2, 3) in beispielsweise
            gewölbter Form (5);

Fig. 5      Ansicht der erfindungsgemäßen Schlinge,
            um den Ureterstein (8) geschlossen, mit
            größerer Breitenabmessung (2) und zueinander geneigten Auflageflächen (7), die
            durch den Schlitz (4) bedingt sind;

Fig. 6      Ansicht der erfindungsgemäßen Schlinge,
            um den Ureterstein (8) geschlossen,
            mit flächiger Auflage der größeren
            Breitenabmessung (2, 3) und zueinander
            geneigten Auflageflächen (7).

Vorrichtung zum Einfangen und Entfernen von Ureter- steinen oder Nierensteinen im Nierenbecken oder im Harnleiter

Patentansprüche

1. Vorrichtung zum Einfangen und Entfernen von Uretersteinen oder Nierensteinen, die sich im Nierenbecken oder im Harnleiter befinden, mit einem Rohr (1) aus biegsamem Material, dessen Endteil (A) nach dem Einführen der Vorrichtung in das Nierenbecken oder in den Harnleiter und Vorbeiführen am Stein zu einer Schlinge umgelegt wird, dadurch g e k e n n - z e i c h n e t , daß der Endteil (A) wenigstens auf einem Teil seiner Länge eine Breitenabmessung (2; 3) hat, die größer als der Außendurchmesser des Rohres (1) ist.

2. Vorrichtung nach Anspruch 1, dadurch g e k e n n - z e i c h n e t , daß die größere Breitenabmessung (2; 3) durch Abflachung des Rohres (1) gebildet ist.

3. Vorrichtung nach Anspruch 2, dadurch g e k e n n - z e i c h n e t , daß in dem abgeflachten Bereich wenigstens eine Schlitzöffnung (4) gebildet ist.

4. Vorrichtung nach Anspruch 2 oder 3, dadurch g e k e n n z e i c h n e t , daß die größere Breitenabmessung (2; 3) zusätzlich durch seitliches Dehnen des Rohrmaterials gebildet ist.

5. Vorrichtung nach Anspruch 3, dadurch g e k e n n - z e i c h n e t , daß die Schlitzöffnung (4) in Längs- oder Querrichtung angeordnet ist.

6. Vorrichtung nach einem der Ansprüche 2 bis 5, dadurch g e k e n n z e i c h n e t, daß die Abflachung gewölbt (5) ist.

7. Vorrichtung nach einem der Ansprüche 2 bis 5, dadurch g e k e n n z e i c h n e t , daß die Abflachung eben ist.

8. Vorrichtung nach einem der Ansprüche 1 bis 7, dadurch g e k e n n z e i c h n e t , daß die größere Breitenabmessung aus dem Material des Rohres (1) gebildet ist.

9. Vorrichtung nach einem der Ansprüche 1 bis 7, dadurch g e k e n n z e i c h n e t , daß die größere Breitenabmessung aus einem anderen Material gebildet ist.

10. Vorrichtung nach einem der Ansprüche 1 bis 9, dadurch g e k e n n z e i c h n e t , daß das Rohr aus Kunststoff gebildet ist.

11. Vorrichtung nach einem der Ansprüche 1 bis 9, dadurch g e k e n n z e i c h n e t , daß das Rohr aus Metall gebildet ist.

12. Vorrichtung nach einem der Ansprüche 1 bis 9, dadurch g e k e n n z e i c h n e t , daß das Rohr (1) sowie das Endteil (A) aus einem Material bestehen, das durch Röntgenstrahlen und/oder Ultraschall geortet werden kann.

13. Vorrichtung nach einem der Ansprüche 1 bis 12, dadurch g e k e n n z e i c h n e t , daß der Endteil (A) mit dem Rohr (1) einstückig ausgebildet ist und weiche Übergänge besitzt.

14. Vorrichtung nach einem der Ansprüche 1 bis 12, dadurch g e k e n n z e i c h n e t , daß der Endteil (A) getrennt gebildet und mit dem Rohr (1) verbunden ist.

0195444

15. Vorrichtung nach einem der Ansprüche 1 bis 14, dadurch g e k e n n z e i c h n e t , daß zur Bildung der Schlinge ein Faden (9) vorgesehen ist, der sich durch das Rohr (1) erstreckt, an einer Stelle vor dem Endteil (A) austritt und mit dem freien Rohrende verbunden ist.

16. Vorrichtung nach einem der Ansprüche 1 bis 15, dadurch g e k e n n z e i c h n e t , daß in dem Bereich größerer Breitenabmessung (2; 3) ein Verstärkungselement (6) angeordnet ist.

- 1/1 -

0195444

Fig. 3

Fig. 4

Fig. 5

Fig. 6

Fig. 1

Fig. 2